(19) Europäisches Patentamt

European Patent Office

Office européen des brevets

(11) **EP 1 598 072 A1**

(12) **EUROPEAN PATENT APPLICATION**
published in accordance with Art. 158(3) EPC

(43) Date of publication:
**23.11.2005 Bulletin 2005/47**

(51) Int Cl.7: **A61K 33/24**, A61P 1/04,
A61P 3/06, A61P 9/00,
A61P 1/10, A61P 17/00,
A61P 25/00, A61P 25/16,
A61P 25/28, A61P 29/00,
A61P 43/00

(21) Application number: **04712230.4**

(22) Date of filing: **18.02.2004**

(86) International application number:
**PCT/JP2004/001825**

(87) International publication number:
**WO 2004/073723 (02.09.2004 Gazette 2004/36)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **20.02.2003 JP 2003042452
28.03.2003 JP 2003090198
27.11.2003 JP 2003396552
09.12.2003 JP 2003409808**

(71) Applicant: **Shetech Co., Ltd.
Tokyo 150-0013 (JP)**

(72) Inventors:
• **MIYAMOTO, Yuse
Tokyo 1540002 (JP)**
• **YOSHIDA, Hideaki
Ibaraki 3001281 (JP)**
• **KAJITA, Masashi
Tokushima 7793125 (JP)**
• **SHIMIZU, Hiroshi
Sapporo-shi, Hokkaido 0640953 (JP)**
• **SHIMIZU, Tadamichi
Sapporo-shi, Hokkaido 0620905 (JP)**

(74) Representative:
**Sternagel, Fleischer, Godemeyer & Partner
Patentanwälte,
An den Gärten 7
51491 Overath (DE)**

(54) **MEDICAL DRUG CONTAINING FINE PARTICLE OF NOBLE METAL**

(57) A medicament for prophylactic and/or therapeutic treatment of a disease selected from the group consisting of a neurodegenerative disease such as amyotrophic lateral sclerosis and Alzheimer's disease, rheumatic disease such as rheumatoid arthritis, ischemic heart disease such as myocardial infarction, stress ulcer, dermatitis, arteriosclerosis and hyperlipidemia, which comprises fineparticles of a noble metal such as platinum or an alloy containing a noble metal (e.g., platinum colloid) as an active ingredient.

**EP 1 598 072 A1**

## Description

Technical Field

The present invention relates to a medicament comprising noble metal fineparticles for prophylactic and/or therapeutic treatment of a neurodegenerative disease such as amyotrophic lateral sclerosis, rheumatic disease, ischemic heart disease, stress ulcer, dermatitis, arteriosclerosis and hyperlipidemia.

Background Art

Amyotrophic lateral sclerosis (hereinafter in the specification, also abbreviated as "ALS") is a progressive neurodegenerative disease in which the upper motor neutrons from the cerebral cortex leading to the spinal cord and the lower motor neurons from the spinal cord leading to the muscles are selectively disturbed. A high onset frequency of ALS is observed, and there are a lot of patients also in our country. Clinically, the disease is characterized by muscular atrophy or muscular weakness, and when clinical stage advances, speech disorder, dysphagia, respiratory disorder and the like are caused by the muscular weakness. Progress of the pathological conditions is relatively fast, and most patients will die in two to four years if an artificial respirator or the like is not used. However, no radical therapy for ALS has been developed, and hence this disease as well as cares of patients has become a great social problem.

ALS is classified into sporadic ALS and familial ALS from a viewpoint of mechanisms of onset. Dominant and recessive inheritances are known for the familial ALS. In recent years, Cu/Zn superoxide dismutase (copper and zinc superoxide dismutase, SOD1), which is a metabolic enzyme for reactive oxygen species, has been identified as a causative gene of the dominant hereditary ALS type 1 (ALS1). Most of ALS patients are classified into sporadic ALS, and a rate of the hereditary ALS is significantly less than 20%. A rate of ALS1 patients relative to total ALS patients is below 2%. Accordingly, discovery of an ALS causative gene other than SOD1 has been desired for elucidation of molecular mechanism of ALS onset and development of therapies. ALS2CR6 gene has been newly isolated and identified as a causative gene of ALS type 2 that involves recessive hereditary mechanism. In addition, concerning the mechanism for generation of reactive oxygen species in neurodegenerative diseases such as ALS, a theory explaining that proteins modified by mutation and the like will aggregate to trigger the generation of reactive oxygen species has been widely supported (Current Topic in Medical Chemistry, 1:507-517, 2001).

For ALS, no radical therapy including pharmacotherapy has been established so far. As for medicaments, riluzole ("Rilutek") has been used as a drug for delaying advance of ALS. Besides the above drug, muscle relaxants, painkillers, tranquilizers, hypnotic agents, vitamin B agents and the like have been used. However, pharmacotherapies using any of these drugs are no more than symptomatic therapies.

Rheumatic diseases are characterized by various anomalies resulting from inflammation or degeneration of connective tissues or metabolic disturbances, and are accompanied by pain and stiffness due to disturbance of motile organs such as joints, muscles, bones, and ligaments. A typical rheumatic disease is rheumatoid arthritis. Systemic erythematodes (SLE), which is a collagen disease in which changes occur in connective tissues of the whole body, and autoimmune diseases are also included in the rheumatic diseases.

Because radical treatment for rheumatoid arthritis (RA) has not yet been developed, empirical symptomatic therapy is still prevailing. Even though therapeutic methods other than pharmacotherapies, e.g., basal therapies (rest, education for patients, physiotherapy and the like), surgical operations and the like have been applied, pharmacotherapy is considered to be the primary medical treatment. As one of the pharmacotherapies, non-steroidal anti-inflammatory agents (NSAID) such as aspirin, indomethacin, and diclofenac have been widely used to reduce pain via suppression of synovitis due to RA. Steroidal agents such as prednisolone may be also used for serious clinical cases. Antirheumatics have also been used to delay advance of osteoclasis by improving immunopathy in RA. As antirheumatics, for example, immunosuppressants such as gold preparations (gold sodium malate, auranofin and the like) and methotrexate have been used.

Cardiac dysfunctions caused by decrease of coronary blood flow due to occurrence of stenosis or obstruction of vessels, resulting from pultaceous hardening of the coronary artery of the heart or the like, are called as ischemic heart diseases, because they cause an ischemic state to cardiac muscles. Typical diseases among the ischemic heart diseases are angina pectoris and myocardial infarction. In myocardial infarction, a thrombus generates continuous ischemia to cause myocardial necrosis, which is sometimes fatal. In milder transient angina pectoris, severe pectoralgia is caused upon attack.

For the pharmacotherapies of angina pectoris, nitrous acid agents such as amyl nitrite, nitroglycerin, and isosorbide dinitrate, and coronary vasodilators such as $\beta$-blockers, calcium antagonists, and dipyridamole are used. For myocardial infarction, peripheral vasodilators, thrombolytic agents such as urokinase, anticoagulants such as heparin sodium, anti-platelet agents such as aspirin and ticlopidine and the like are used for an acute stage. However, no medicament has been provided that can effectively suppress myocardial necrosis especially in an acute stage of myocardial infarction.

Applications of various medicaments have been attempted also for stress ulcer, dermatitis, arteriosclerosis, and hyperlipidemia. However, almost no medicament that successfully achieves a satisfactory curative effect has been provided, and therefore, development of novel medicaments has been desired.

It is known that platinum colloid scavenges hydrogen peroxide, which is one of reactive oxygen species (for example, Japanese Patent Unexamined Publication (KOKAI) No. 10-68008, paragraph 0040). However, the aforementioned patent publication does not teach efficacy of platinum colloid in therapeutic and/or prophylactic treatment of a neurodegenerative disease such as ALS, rheumatic disease, ischemic heart disease, stress ulcer, dermatitis, arteriosclerosis, and hyperlipidemia.

Disclosure of the Invention

An object of the present invention is to provide a medicament for prophylactic and/or therapeutic treatment of a neurodegenerative disease such as ALS and Alzheimer's disease, rheumatic disease such as rheumatoid arthritis, ischemic heart disease such as myocardial infarction, stress ulcer, dermatitis, arteriosclerosis, and hyperlipidemia. The inventors of the present invention conducted various researches to achieve the aforementioned object, and as a result, they found that prophylactic and/or therapeutic treatment for each of the aforementioned diseases was successfully achieved by administering metal colloid such as platinum colloid. The present invention was achieved on the basis of the aforementioned finding.

The present invention thus provides a medicament for prophylactic and/or therapeutic treatment of a disease selected from the group consisting of a neurodegenerative disease, a rheumatic disease, an ischemic heart disease, stress ulcer, dermatitis, arteriosclerosis, and hyperlipidemia, which comprises fineparticles of a noble metal or an alloy containing a noble metal as an active ingredient.

According to preferred embodiments, the present invention provides the aforementioned medicament, wherein the neurodegenerative disease is amyotrophic lateral sclerosis, Alzheimer's disease, or Parkinson's disease; the aforementioned medicament, wherein the neurodegenerative disease is amyotrophic lateral sclerosis; the aforementioned medicament, wherein the rheumatic disease is rheumatoid arthritis; the aforementioned medicament, wherein the ischemic heart disease is myocardial infarction at an acute stage; and the aforementioned medicament, wherein the stress ulcer is gastric stress ulcer or duodenal stress ulcer.

According to more preferred embodiments, the present invention provides the aforementioned medicament, wherein the noble metal consists of one or more kinds of noble metals selected from the group consisting of ruthenium, rhodium, palladium, and platinum; the aforementioned medicament, wherein the noble metal is platinum; and the aforementioned medicament, wherein the fineparticles of a noble metal consist of platinum colloid having a mean particle size of 10 nm or smaller.

From another aspect, the present invention provides a method for prophylactic and/or therapeutic treatment of a disease selected from the group consisting of a neurodegenerative disease, rheumatic disease, ischemic heart disease, stress ulcer, dermatitis, arteriosclerosis, and hyperlipidemia, which comprises the step of administering fineparticles of a noble metal to a mammal including human. The present invention also provides use of fineparticles of a noble metal for manufacture of the aforementioned medicament.

Brief Explanation of the Drawings

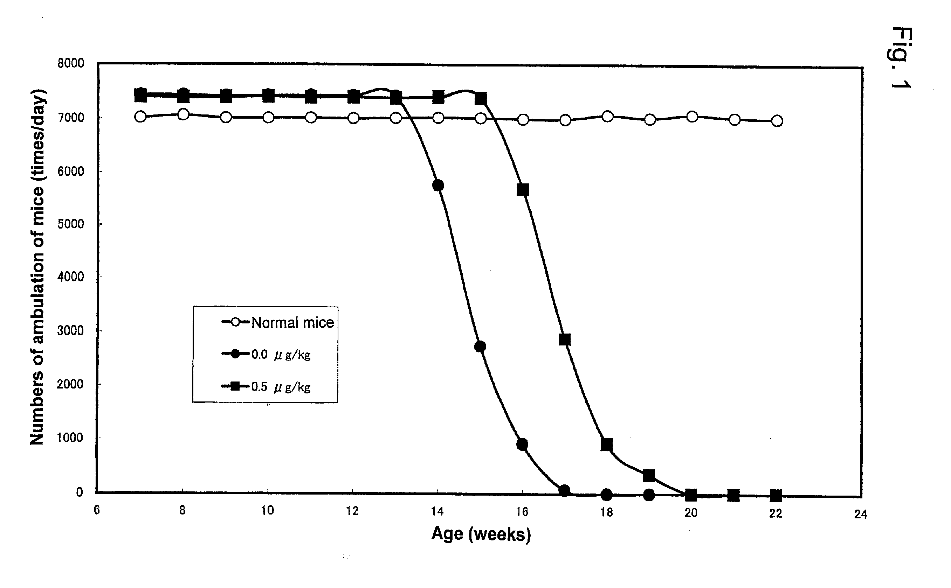

Fig. 1 shows the results obtained by administering the medicament of the present invention to amyotrophic lateral sclerosis model mice and measuring momentum of the mice using an infrared sensor. ○ represents the results for normal mice, ■ represents the results for the group administered with the medicament of the present invention (0.5 μ g/kg), and ● represents the results for the group not administered with the medicament of the present invention (pathological mice).

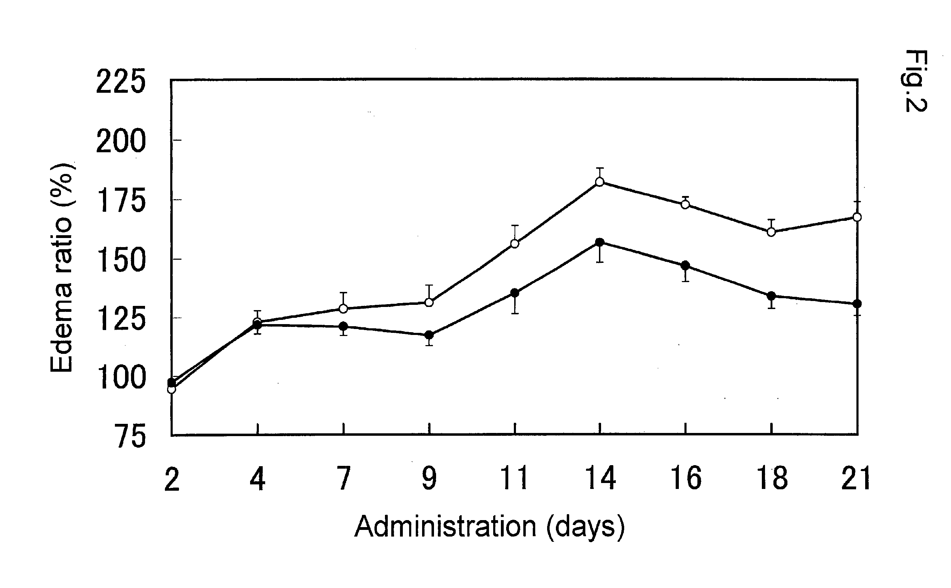

Fig. 2 shows the effect of the medicament of the present invention on the edema ratio obtained in Example 4. ○ represents the results for the control group (physiological saline-administered group), and ● represents the results for the group administered with the medicament of the present invention (5 μ mol/kg/day).

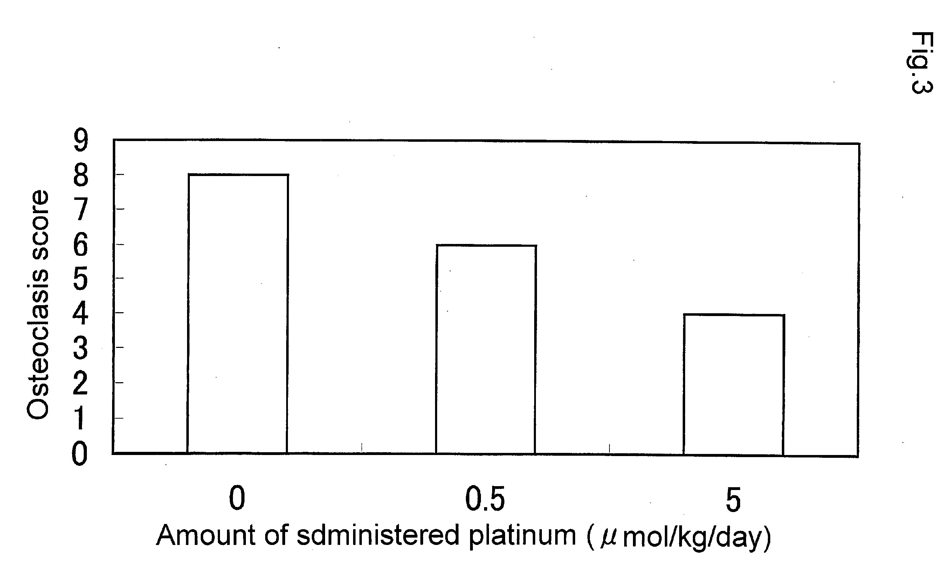

Fig. 3 shows the efficacy of the medicament of the present invention on osteoclasis obtained in Example 4.

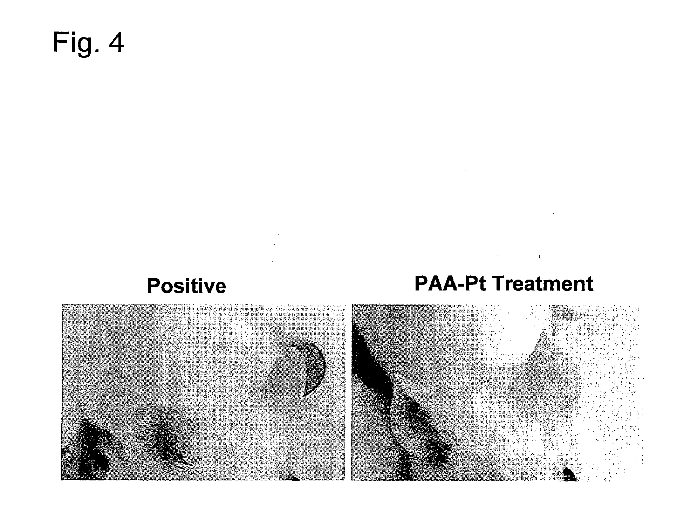

Fig. 4 comprises photographs showing the condition of auricula of mouse on the tenth day after the UVA irradiation (20 $J/cm^2$) observed in Example 7. The photograph on the left side indicates the result for the positive control, and the photograph on the right side indicates the result of the mouse applied with the medicament of the present invention.

Best Mode for Carrying out the Invention

The medicament of the present invention is used for prophylactic and/or therapeutic treatment of a disease selected from the group consisting of a neurodegenerative disease, rheumatic disease, ischemic heart disease, stress ulcer, dermatitis, arteriosclerosis, and hyperlipidemia, and is characterized to comprise fineparticles of a noble metal as an active ingredient. Types of the noble metal are not particularly limited, and any of gold, ruthenium, rhodium, palladium, osmium, iridium, and platinum may be used. However, preferred noble metals are ruthenium, rhodium, palladium, and platinum. The fineparticles of noble metal may contain two or more kinds of noble metals. Fineparticles of an alloy containing at least one kind of noble metal, or a mixture containing fineparticles of one or more kinds of noble metals and fineparticles of one or more kinds of metals other than noble metal can also be used. For example, an alloy comprising gold and platinum or the like may be used. Among them, platinum or an alloy containing platinum is preferred, and platinum is particularly preferred.

As the fineparticles of a noble metal, fineparticles that have a large specific surface area and can form a colloidal state of superior surface reactivity are preferred. A particle size of the fineparticles is not particularly limited. Fineparticles having a mean particle size of 50 nm or smaller can be used, and fineparticles having a mean particle size of, preferably 20 nm or smaller, further preferably 10 nm or smaller, most preferably about 1 to 6 nm, can be used. Fineparticles having a smaller particle size can also be used.

Various preparation methods for noble metal fineparticles are known (for example, Japanese Patent Publication (KOKOKU) Nos. 57-43125, 59-120249, Japanese Patent Unexamined Publication Nos. 9-225317, 10-176207, 2001-79382, 2001-122723 and the like), and those skilled in the art can easily prepare the fineparticles by referring to these methods. For example, as the method for producing noble metal fineparticles, a chemical method called precipitation method or metal salt reduction method, or a physical method called combustion method can be used. Fineparticles prepared by any of the methods may be used as the active ingredient of the medicament of the present invention. It is preferable to use fineparticles prepared by the metal salt reduction method from viewpoints of easiness of the production and quality of the fineparticles.

In the metal salt reduction method, for example, an aqueous solution or organic solvent solution of a water-soluble or organic solvent-soluble noble metal salt or noble metal complex is prepared, then a water-soluble polymer is added to the solution and pH of the solution is adjusted to 9 to 11, and the solution can be refluxed by heating in an inert atmosphere to reduce the metal salt or metal complex to obtain metal fineparticles. Types of the water-soluble or organic solvent-soluble noble metal salt are not particularly limited. For example, acetate, chloride, sulfate, nitrate, sulfonate, phosphate and the like can be used, and complexes thereof may also be used.

Types of the water-soluble polymer used for the metal salt reduction method are not particularly limited. For example, polyvinylpyrrolidone, polyvinyl alcohol, polyacrylic acid, cyclodextrin, amiropectin, methylcellulose and the like can be used, and two or more kinds of these polymers may be used in combination. Polyvinylpyrrolidone can be preferably used, and poly(1-vinyl-2-pyrrolidone) can be more preferably used. It is also possible to use various kinds of surface active agents such as anionic, nonionic or liposoluble surface active agents instead of the water-soluble polymer or together with the water-soluble polymer. When an alcohol is used to perform the reduction, ethyl alcohol, n-propyl alcohol, n-butyl alcohol, n-amyl alcohol, ethylene glycol or the like is used. However, the methods for preparing noble metal fineparticles are not limited to the methods explained above.

The medicament of the present invention can be used for prophylactic and/or therapeutic treatment of a disease selected from the group consisting of a neurodegenerative disease, rheumatic disease, ischemic heart disease, stress ulcer, dermatitis, arteriosclerosis, and hyperlipidemia.

Examples of the neurodegenerative disease include, for example, amyotrophic lateral sclerosis, Alzheimer's disease, Parkinson's disease, and the like. However, the diseases are not limited to these examples. A preferred disease for application of the medicament of the present invention includes amyotrophic lateral sclerosis.

Examples of the rheumatic disease include, for example, rheumatoid arthritis, juvenile rheumatoid arthritis, erythematosus (discoid lupus erythematosus, systemic erythematodes, drug-related lupus erythematosus, and the like), pachydermia, diffuse fasciopasy, polymyositis, necrotizing angitis and other angiopathies, diffuse connective tissue disorders such as Sjoegren's syndrome and overlap syndrome, arthritis accompanied by myelitis, degenerative arthritis (osteoarthropathy, osteoarthritis), arthritis accompanying contagium, metabolic and endocrinologic diseases accompanied by rheumatic symptoms, neoplasm (tumor), nervous and vascular anomaly, bone disease and chondropathy, extraarticular disease, various diseases with arthrosis, and the like (Decker J.L. et al., Arth. Rheum., 26 (8), 1983). However, the diseases are not limited to these examples. A preferred disease for application of the medicament of the present invention includes rheumatoid arthritis.

The term "ischemic heart disease" used in the specification encompasses at least angina pectoris and myocardial infarction, and encompasses various pathological types of each disease. For example, angina pectoris includes exertional angina (effort angina), spontaneous angina (angina at rest), and the like ("Angina Pectoris, $\beta$ -blockers - Clinical Pharmacology and Clinical Applications", edited by Ebihara et al., Clinical Medicine Research Association, 1989), and the disease may also be classified into exertional angina and unstable angina (American Health Association). In myocardial infarction, obstruction generally occurs in a large branching of the coronary artery, and necrosis arises over a wide region in the perfusion region. Ischemic heart diseases may also be classified into exertional angina, myocardial infarction (including acute and old myocardial infarctions), intermediate, and indolent ischemic heart diseases (including subclinical and chronic myocardiopathies) ("Angina Pectoris", edited by Abe et al., Kanehara Shuppan, 1985). It is known that vascular restenosis or reocclusion occurs at a high rate after a balloon or catheter treatment in PTCA (Percutaneous Transluminal Coronary Angioplasty), which is performed as a therapeutic treatment of myocardial infarction. Heart disorders accompanied by vascular restenosis or reocclusion caused by these treatments are also included in the ischemic heart diseases. The term "ischemic heart disease" used in the specification should be construed in the broadest sense so as to include all of these diseases, and should not be construed in any limitative way.

The stress ulcer includes peptic ulcer, and more specifically, the disease includes gastric ulcer and duodenal ulcer. It is known that stresses serve as a primary cause of peptic ulcer as an exogenous cause. The medicament of the present invention can be applied to ulcer of which major cause is a stress. The medicament of the present invention can be applied when participation of a stress is suspected, as well as when a causative stress is definite.

Dermatitis includes, for example, contact dermatitis caused by contact with a toxic chemical substance or light (primary irritant contact dermatitis, allergic contact dermatitis, phototoxic contact dermatitis, photoallergic contact dermatitis, and the like, (Handbook of Dermatological Treatment, edited by Okido, Nanzando, 1989) as well as eczemas (acute eczema and chronic eczema), atopic dermatitis (atopic dermatitis in neonatal period to babyhood, infancy to later childhood and adulthood, and the like), seborrheic dermatitis, autosensitisation dermatitis, drug eruption, and the like. The medicament of the present invention can be systemically administered, or can be topically administered to a dermatitis site.

Arteriosclerosis is a general term for arterial lesions with reconstruction, sclerosis, and hypofunction of arterial walls, and includes pathological conditions of medial sclerosis, arteriocapillary sclerosis, pultaceous sclerosis (atherosclerosis), and the like. Arteriosclerosis treatable by the medicament of the present invention may be any kind of these diseases. A kind of artery is not particularly limited. For example, artery may be any of coronary artery, cerebral artery, renal artery, appendicular artery, and the like. Arteriosclerosis treatable by the medicament of the present invention may be at any of pathological periods, e.g., conversion of endothelial cells into foam cells at an early stage of arteriosclerosis, necrocytosis of foam cells, and lipid deposition in atherosclerotic lesions. The diseases to be applied by the medicament of the present invention should be construed in the broadest sense so as to include pathological conditions during a processes of formation of arteriosclerosis, as well as already formed arteriosclerosis. Moreover, the medicament of the present invention has a hypolipidemic action in blood, and thus can be used for prophylactic and/or therapeutic treatment of hyperlipidemia.

In the specification, the term of "prophylactic and/or therapeutic treatment" should be construed in its broadest sense including prophylaxis of onsets of the aforementioned diseases and therapeutic treatment of the aforementioned diseases after onsets, as well as suppression of advance of the aforementioned diseases, improvement or amelioration of the aforementioned diseases, prevention of relapse of the aforementioned diseases, and the like, and the term should not be construed in any limitative way.

Administration routes of the medicament of the present invention are not particularly limited, and either route of oral administration or parenteral administration may be chosen. As the medicament of the present invention, noble metal dispersion of a colloidal state or noble metal fineparticles in a dried state prepared by the methods explained above may be used without any treatment. The metal fineparticles prepared in water, organic solvent, or mixture of water and organic solvent exist in a colloidal state. The aforementioned noble metal dispersion in a colloidal state, per se, can be used as the medicament of the present invention. An aqueous suspension in which the noble metal fineparticles associate to form clusters may also be used as the medicament of the present invention. Furthermore, when it is desirable to remove a solvent, the solvent can be removed by an operation of heating or the like to obtain fineparticles in a dried state, and the dried fineparticles obtained by the above operation can be used as the medicament of the present invention. Water containing platinum fineparticles, which is a soft drink (for example, "Hakkin Gensui", Inovex Co., Ltd.), platinum and palladium colloid preparation ("Paplal for internal application", Toyo Kosei Seiyakusho) as a curative agent for acute gastroenteritis or chronic esogastritis and the like can also be used as the medicament of the present invention.

The medicament of the present invention can also be administered as a pharmaceutical composition for oral or parenteral use that can be produced by a method well known to those skilled in the art. Examples of the pharmaceutical composition suitable for oral administration include, for example, tablets, capsules, powders, subtilized granules, granules, solutions, syrups, and the like, and examples of the pharmaceutical composition suitable for parenteral administration include, for example, injections, drip infusions, suppositories, inhalants, eye drops, nasal drops, ear drops, ointments, creams, transdermal preparations, transmucosal preparations, patches, and the like. The aforementioned pharmaceutical compositions can be produced by using one or more kinds of pharmaceutical additives together with the noble metal fineparticles as the active ingredient. Examples of the pharmaceutical additives include, for example, excipients, disintegrating agents or disintegrating aids, binders, lubricants, coating agents, dyes, diluents, bases, dissolving agents or dissolving aids, isotonic agents, pH modifiers, stabilizers, propellants, tackifiers, and the like, and they can be suitably selected by those skilled in the art depending on the dosage form of the pharmaceutical composition.

Doses of the medicament of the present invention are not particularly limited, and the dose can be suitably chosen depending on conditions such as a type of disease, a purpose of administration (prophylactic or therapeutic treatment), an age, body weight, symptoms and the like of a patient. The medicament of the present invention can be used by oral administration for adults in an amount in a range of, for example, about 0.001 to 1,000 mg per day on the basis of a weight of the noble metal fineparticles.

Examples

The present invention will be explained more specifically with reference to the examples. However, the scope of the present invention is not limited to the following examples.

Example 1

In a 100-ml 2-neck pear-shaped flask connected with an allihn condenser and a 3-neck joint, 0.1467 g of poly (1-vinyl-2-pyrrolidone) (Wako Pure Chemical Industries) was placed and dissolved in 23 ml of distilled water. This solution was stirred for 10 minutes then mixed with 2 ml of $1.66 \times 10^{-2}$ M chloroplatinic acid solution obtained by dissolving hexachloroplatinic acid ($H_2PtCl_6 \cdot 6H_2O$, Wako Pure Chemical Industries) in distilled water, and stirred for additional 30 minutes. The inside atmosphere of the reaction system was replaced with nitrogen gas. Twenty five ml of special grade ethanol was added to the reaction mixture and the resulting mixture was refluxed at a temperature of 100°C for 2 hours while the nitrogen atmosphere was maintained. An ultraviolet-visible light spectral scanning analysis of the reaction mixture was performed to confirm disappearance of the platinum ion peak and saturation of the peak due to scattering peculiar to metal solid and thereby confirm completion of the reduction reaction. The organic solvent was evaporated under reduced pressure to prepare a platinum colloidal solution containing platinum fineparticles (mean particle size: $2.4 \pm 0.7$ nm). In the following examples, this platinum colloidal solution is referred to as PVP-Pt. In a similar manner, a platinum colloidal solution having a mean particle size of $2.0 \pm 0.4$ nm was prepared by using sodium polyacrylate (Aldrich, in an amount 125 times relative to Pt in terms of unit weight) instead of the poly(1-vinyl-2-pyrrolidone). In the following examples, this platinum colloidal solution is referred to as PAA-Pt.

Example 2

B6SJL-TgN(SODIG93A)GUr mice of 6- to 8- week old (amyotrophic lateral sclerosis model mice) were bred with ad libitum feeding of 0.66 μ M, 0.066 μ M, and 6.6 nM in the concentration in the aforementioned platinum colloidal solution (PVP-Pt). After the age approximately 16 weeks, typical symptoms of amyotrophic lateral sclerosis were observed in the mice of the control group fed with ordinary water, that is, movement of the hind legs ceased, and the mice began only to crawl by the forelegs. Whilst, in the mice administered with the platinum colloidal solution, improvement of the aforementioned symptoms was observed in a dose-dependent manner. In the mice of the group administered with the 6.6 nM solution, the mice were somehow able to walk, although anomalies such as staggers were observed in the hind legs at the time of walking. In the mice of the group administered with the 0.066 μ M solution, the mice were in a state that they were able to arise rather quickly, although shaking was observed in the hind legs, and in the mice of the group administered with the 0.66 μ M solution, expression of the aforementioned symptoms was not observed, and the mice had the same ambulatory ability as that of normal mice.

Example 3

B6SJL-TgN(SODIG93A)GUr mice of 3-week and 7-week old were administered with 0.5 μ M of the aforementioned platinum colloidal solution (PVP-Pt, the dose is indicated in terms of the dose of platinum), and numbers of ambulation of the mice were counted by using an infrared sensor. A less number of the ambulation means that motions were decreased due to the onset of amyotrophic lateral sclerosis. B6SJL-TgN(SODIG93A)GUr mice administered with water instead of the platinum colloidal solution were used as a comparative group (pathological mice) for comparison with normal mice. The results of the experiment using 7-week old mice are shown in Fig. 1. In the group of mice administered with the medicament of the present invention, the decrease of motions due to the onset of amyotrophic lateral sclerosis was significantly suppressed.

Example 4

Rats (LEW/CrJ rats, 100 to 130 g, 7-week old, Charles River Japan) were used for experiments after quarantine of 5 days and a subsequent habituation period of 8 days. The animals were bred with ad libitum feeding of pellets (CRF-1, Oriental Yeast) under the conditions of a room temperature of 20 to 26°C, humidity of 40 to 70% and light and darkness each for 12 hours. An inflammatory agent *(Mycobacterium butyricum,* Difco) was suspended in liquid paraffin at a concentration of 1.0 mg/0.1 mL, and administered into the left hind footpads under anesthesia by using a disposable polypropylene syringe attached with a 27G hypodermic needle (Mantoux needle). The inflammatory agent was administered at a dose of 0.1 mL/animal before the administration of the medicament on the day of the start of the administration of the medicament.

As the medicament of the present invention, the aforementioned platinum colloidal solution (PAA-Pt) was administered to the caudal veins using a disposable polypropylene syringe attached with a 25G hypodermic needle. The administration frequency was once a day, and the administration period was 20 days from the day of the start of the administration of the medicament as being the first day. The doses were 0.05 μ mol/kg, 0.5 μ mol/kg and 5 μ mol/kg. As a control, physiological saline was similarly administered into the caudal veins.

Each group consisted of ten animals, and the volumes of hind footpads on the both sides were measured by using a plethysmometer (footpad edema volumetric apparatus, TK-101CMP, Unicom) before the administration of the medicament on the 1st, 2nd, 4th, 7th, 9th, 11th, 14th, 16th, and 18th administration days and the day of autopsy. From the footpad volumes of the first administration day and each measurement day, the edema ratio was calculated by using the following equation:

Edema ratio (Δ %) = (Footpad volume (mL) on each measurement day - Footpad

volume (mL) on the 1st administration day) ÷ Footpad volume on the 1st administration day

(mL) × 100.

On the day of autopsy, the animals were sacrificed by bleeding from the ventral aorta under anesthesia, and the both hind legs were cut at the center of the thighbone and fixed with neutrally buffered 20% formalin. After the fixation, the legs were photographed by soft X-ray photography by using a soft X-ray system (SOFROM SRO-505C, SOFROM). Osteoclasis was observed on a soft X-ray film projected by a projector, and the degree of osteoclasis was represented with scores. Scoring was performed for the calcaneus, tarsalia, metatarsus, and tibia according to the following judgment criteria, and the total score was calculated for the four kinds of bones. Osteoclasis Score
0: Normal
1: Mild osteoclasis and decrease of bone density
2: Intermediate osteoclasis and decrease of bone density
3: Severe osteoclasis and decrease of bone density

The effect of the medicament of the present invention on the edema ratio is shown in Fig. 2. The efficacy of the medicament of the present invention on osteoclasis is shown in Fig. 3. It is clearly understood that the medicament of the present invention has a superior therapeutic effect on edema and osteoclasis. In the pathological findings, remarkable improvement was observed for the medicated group compared with the control group in tissue destruction radiograms of edema, abscess, osteoclasis and the like, although no difference in infiltration of inflammatory cells into the pathological sites was observed between the medicated group and the control group.

Example 5

After quarantine of 5 days and a habituation period of 2 days or more, measurement of body weight and observation of general conditions of rabbits (Kb1:JW(SPF) rabbits, 2.00 to 2.80 kg, 12-week old, Kitayama Labes) were performed, and animals for which abnormality was not observed in the general conditions and the weight increase were used for experiments. The animals were bred with feeding of 100 g per day of pellets (RC-4, Oriental Yeast) under the conditions of a room temperature of 20 to 26°C, humidity of 40 to 70% and light and darkness each for 12 hours.

The rabbits of 13- to 16-week old were each anesthetized by administering 30 mg/mL/kg of pentobarbital sodium from the auricular vein and then fixed in the dorsal position. A tracheal cannula was inserted into the trachea and then connected to an artificial respirator (141A, NEW ENGLAND INSTRUMENTS INC., setting conditions: 40 to 60 times/minute, 20 to 30 mL/time, adjusted in these ranges depending on the anesthetization condition) to maintain respiration. The blood pressure was introduced into an amplifier for distorted pressure (AP-601G, Nihon Kohden) and a hemodynamometry unit (AP-611G, Nihon Kohden) via a pressure transducer (TP-300T, Nihon Kohden) connected to an arterial cannula inserted into the femoral artery and recorded on a recorder (WT-645G, Nihon Kohden). An electrocardiogram (second induction) was introduced into an amplifier for bioelectricity (AB-621G, Nihon Kohden) via a needle electrode and recorded on a recorder (WT-645G, Nihon Kohden).

The treated rabbits were each subjected to thoracotomy of excision between the left fourth and fifth ribs. After excision of the cardiac pyogenic membrane, the heart was exposed out of the thorax, and the left coronary artery branch (LCA) was ligated using a suture with a needle (3/8 circular round needle, NIPPON SHOJI KAISHA, LTD.) to close the artery for 30 minutes (ischemia). Thirty minutes after the closing, the ligated suture was loosened (the suture was left in the thoracic cavity to facilitate immediate ligation of LCA) to open the vessel (reperfusion). After sixty minutes, the chest was closed, and the animal was returned into the rearing room.

About 48 hours after the opening, the animals were anesthetized with pentobarbital in the same manner as described above and then sacrificed by bleeding from the common carotid arteries. The animals were each subjected to thoracotomy, and the heart was extracted and washed with physiological saline. LCA of the washed heart was ligated and stained by perfusion from an incised opening of aorta with about 1 to 1.5 mL of 0.5% Evans Blue. After the staining, only the left ventricle was isolated from the heart, and the cardiac muscles were sliced form the cardiac head at intervals of 5 mm (6 slices for each animal), put into a beaker containing 1% TTC phosphate buffer, and warmed for 10 minutes in an incubator set at 37°C. After the warming, the sliced cardiac muscles were photographed and divided by cutting into the non-ischemic region (Evans Blue-stained region, A), ischemic non-infarction region (TTC-stained region in the non-Evans Blue-stained region, B), and infarction region (non-TTC-stained region in the non-Evans Blue-stained region, C), and weight of each was measured. From the measured weights, the ratio of the weight of the ischemic regions (total of B + C for 6 slices) relative to the weight of the total left ventricle (total of A + B + C for 6 slices) and the ratio of the weight of the infarction regions (total of C for 6 slices) relative to the weight of the ischemic regions were calculated. The 1% TTC phosphate buffer was prepared by dissolving TTC at a concentration of 1 w/v% with a phosphate buffer obtained by dissolving a phosphate buffer tablet in water for injection. The 0.5% Evans Blue was prepared by dissolving Evans Blue with water for injection at a concentration of 0.5 w/v%.

PAA-Pt was diluted with physiological saline and then intravenously administered. The medicament was administered from the femoral vein (0.5 μ g/kg) as a single dose from 5 minutes before the reperfusion, and persistently administered until the reperfusion was completed (0.5 μ g/kg/hr). The administration volume was 0.1 mL/kg for the single dose administration, and 1 mL/kg/hr for the continued administration. Further, 24 hours after the end of the reperfusion, the medicament was administered again as a single dose administration. As a control, physiological saline was administered by single dose administration and continued administration in the same manner as that used for the aforementioned medicated group (administration volume was 0.1 mL/kg for the single dose administration, and 1 mL/kg/hr for the continued administration). The results are shown in Table 1. From the results shown in Table 1, it is clearly understood that the medicament of the present invention significantly decreased the infarction regions and ischemia regions in the myocardial ischemia reperfusion disturbance model, and that highly remarkable effect was observed especially for the 5 μ mol/kg-administered group.

Table 1

| Group | Animal number | Infarction amount (g) | Infarction + ischemia amount (g) | Left ventricle amount (g) | Infarction area (%) | Ischemic area (%) |
|---|---|---|---|---|---|---|
| Vehicle (Physiological saline) | 101 | 0.94 | 2.17 | 5.43 | 43.3 | 40.0 |
| | 102 | 0.60 | 2.16 | 5.19 | 27.8 | 41.6 |
| | 104 | 1.20 | 2.09 | 5.46 | 57.4 | 38.3 |
| | 106 | 1.05 | 2.13 | 6.28 | 49.3 | 33.9 |
| | 107 | 1.09 | 2.09 | 5.41 | 52.2 | 38.6 |
| | 108 | 1.00 | 2.88 | 6.15 | 34.7 | 46.8 |
| | 109 | 0.49 | 1.66 | 5.65 | 29.5 | 29.4 |
| | 111 | 0.77 | 1.47 | 5.19 | 52.4 | 28.3 |
| | 113 | 0.63 | 1.89 | 5.47 | 33.3 | 34.6 |
| | Average | | | | 42.2 | 36.8 |
| | standard error | | | | 3.7 | 2.0 |

Table 1 (continued)

| Group | Animal number | Infarction amount (g) | Infarction + ischemia amount (g) | Left ventricle amount (g) | Infarction area (%) | Ischemic area (%) |
|---|---|---|---|---|---|---|
| PAA-Pt 0.5 μ mol/kg | 201 | 0.73 | 1.57 | 5.15 | 46.5 | 30.5 |
| | 202 | 0.99 | 2.46 | 5.31 | 40.2 | 46.3 |
| | 205 | 0.28 | 1.92 | 5.08 | 14.6 | 37.8 |
| | 206 | 0.61 | 1.37 | 5.35 | 44.5 | 25.6 |
| | 207 | 0.46 | 2.19 | 5.30 | 21.0 | 41.3 |
| | Average | | | | 33.4 | 36.3 |
| | standard error | | | | 6.5 | 3.7 |
| PAA-Pt 5 μ mol/kg | 301 | 0.11 | 2.05 | 5.94 | 5.4 | 34.5 |
| | 302 | 0.82 | 2.22 | 5.38 | 36.9 | 41.3 |
| | 303 | 0.28 | 2.23 | 6.28 | 12.6 | 35.5 |
| | 304 | 0.68 | 2.77 | 6.17 | 24.5 | 44.9 |
| | 305 | 0.78 | 2.14 | 5.12 | 36.4 | 41.8 |
| | 306 | 0.34 | 1.77 | 4.84 | 19.2 | 36.6 |
| | 307 | 1.65 | 2.42 | 5.17 | 68.2 | 46.8 |
| | 308 | 0.40 | 2.57 | 5.92 | 15.6 | 43.4 |
| | 309 | 0.78 | 2.39 | 5.59 | 32.6 | 42.8 |
| | 310 | 0.13 | 1.22 | 5.84 | 10.7 | 20.9 |
| | Average | | | | 26.2* | 38.9 |
| | standard error | | | | 5.8 | 2.4 |

*$p < 0.05$: Significant difference relative to vehicle (Student's t-test)

Example 6

Rats (Crj:CD(SD)IGS rats, 140 to 210 g, male, 6-week old, Charles River Japan) were used for experiments after quarantine of 5 days and a subsequent habituation period of 2 days. The animals were bred with ad libitum feeding of pellets (CRF-1, Oriental Yeast) under the conditions of a room temperature of 20 to 26°C, humidity of 40 to 70% and light and darkness each for 12 hours. As the medicament of the present invention, the aforementioned platinum colloidal solution (PAA-Pt) was administered from the caudal vein using a disposable polypropylene syringe attached with a 25G hypodermic needle (2 mL/kg) or orally administered (5 mL/kg) once a day. As a control, physiological saline was similarly administered into the caudal vein.

The sample was administered, and after 30 minutes, the animals were put into a stainless steel cage for restraint (4.5 × 4.5 × 18 cm, 10-compartment cage) and immersed in a water tank of 23 ± 1°C so that the animal was immersed in water up to the thorax xiphisternum thereof. After the submersion restraint of 7 hours, the rats were euthanized by cervical dislocation, and the stomach was extracted from each animal. In the inside of the extracted stomach, 10 mL of physiological saline was filled, and the stomach was further immersed in 1% formalin and fixed until the next day. After the fixation, the stomach was excised along the greater curvature and lightly washed with physiological saline, and then the length of ulcer was measured. The major axis was measured among the minor axis and major axis, and the total was used as a value for that animal.

Average ± standard error (mm) of the major axis of ulcer was calculated for every group. As for significance test, the results were compared between the medium-administered group and the PAA-Pt-administered group for each administration route. After the results were subjected to F-test, Student's t-test was performed when homoscedasticity was observed, and Aspin-Welch's t-test was performed when heteroscedasticity was observed. As for the significance level, the level of less than 5% is considered to be significant, and the results with the level of less than 5% ($p < 0.05$)

or those of less than 1% (p<0.01) were separately indicated. After the restraint of 3 hours, the diameter of ulcer (average) of the physiological saline-administered group was 19.86 mm (standard deviation: 8.41 mm), and the diameter of ulcer (average) of the PAA-Pt-administered group was 7.27 mm (standard deviation: 2.57 mm, p = 0.00566353). Further, after the restraint of 7 hours, the diameter of ulcer (average) of the physiological saline-administered group was 43.88 mm (standard deviation: 11.96 mm), and the diameter of ulcer (average) of the PAA-Pt-administered group was 19.84 mm (standard deviation: 7.50 mm, p = 0.00191338).

Example 7

BALB/c mice were used as groups each consisting of 4 animals to examine the efficacy of the medicament of the present invention on dermatitis caused by photosensitization using lomefloxacin (LFLX, known to cause photosensitization) which is a new quinolone class synthetic antibacterial agent (Tokura, Y. et al., J. Immunol., 160, pp. 3719-3728, 1998; Watanabe, H., et al., J. Biol. Chem., 279, pp.1676-1683, 2004). The mice administered with 2 mg/0.2 ml of LFLX (i.p.) were irradiated with UVA ($12 \text{ J/cm}^2$) on a abdominal shaved area to cause photosensitization. A gel ointment was prepared by adding 1 mM of PAA-Pt to an aqueous solution containing 2% of carboxyvinyl polymer and applied to the both sides of auricula in an amount of 0.3 g/ear from the next day of the sensitization to the 5th day (applied after photoirradiation on the 5th day). For a positive control, only 2% (w/w) carboxyvinyl polymer was applied. On the 5th day, 2 mg/0.2 ml of LFLX was intraperitoneally administered (i.p.), and the both auriculas were irradiated with UVA ($20 \text{ J/cm}^2$). For the non-sensitization group, only administration (i.p.) of 2 mg/0.2 ml of LFLX and UVA irradiation ($20 \text{ J/cm}^2$) were performed on the 5th day. As a result, in the platinum colloid-applied group, auricular swelling was significantly reduced 24 hours after the irradiation (on the 6th day) compared with the positive control group. When flare was observed on the 10th day from the UVA irradiation ($20 \text{ J/cm}^2$), erythema and swelling of the auriculas were apparently cured in the platinum colloid-applied group (Fig. 4).

Table 2

| Mouse group | Auricular swelling (Average ± SEM, μ m) |
|---|---|
| Positive control group | 52.5 ± 4.5 |
| PAA-Pt-applied group | 26.9 ± 4.1* |
| Negative control group | 11.3 ± 3.5 |

*: p<0.001 (n = 8)

Example 8

BALB/c mice were used as groups each consisting of 4 to 6 animals and each applied on a back shaved area with 100 μ l of 1% TCSA (3,3,4,5-tetrachlorosalicylanilide, in a mixture of olive oil and acetone (1:4)) on the day of the start of the test and the next day, and the applied area was irradiated with UVA ($16 \text{ J/cm}^2$) to cause photosensitization (Suzuki, K. et al., J. Dermatol. Sci., 23, pp.138-144, 2000). From the next day of the sensitization to the 5th day, 0.3 g/ear of hapten was applied to the both sides of auricula for hapten application on the auricula (on the 5th day, application was performed after the photoirradiation). Mice as positive control were applied only with 2% (w/w) carboxyvinyl polymer. On the 5th day, 40 μ l of 0.1% TCSA was applied to the both earlobes of each mouse, and UVA ($16 \text{ J/cm}^2$) was irradiated from a distance of 40 cm. For the non-sensitization group, only the application of 40 μ l of 0.1% TCSA and irradiation of UVA ($16 \text{ J/cm}^2$) were performed on the 5th day. Twenty-four hours after the irradiation, the thickness of the ear was measured. As a result, the photocontact dermatitis reaction was weaker in the platinum colloid-applied group compared with the positive control group.

Table 3

| Mouse group | Auricular swelling (average ± SEM, μ m) |
|---|---|
| Positive control group (n = 4) | 36.2 ± 5.5 |
| PAA-Pt-applied group (n = 6) | 14.2 ± 4.0* |
| Negative control group (n = 4) | 9.2 ± 3.2 |

*: p<0.05 v.s. Positive control

Example 9

Kbl:JW rabbits (SPF, male, body weight: 1.80 to 2.70 kg, Kitayama Labes) were bred with quarantine of 5 days and a subsequent habituation period of 9 days, and during these periods, body weight was measured 3 times, and general conditions were observed every day. As the feed, pellets (RC4, Oriental Yeast) were used. The animals were then acclimated for 14 days with 0.5% cholesterol-containing feed (feed containing 0.5% of cholesterol, 3% of peanut oil and 3% of coconut oil). Animals of which total cholesterol value favorably rose were selected, and divided into 4 groups each consisting of 10 animals so that the averages of body weight and total cholesterol value were almost the same among the groups on the day of the division of the animals into the groups. The animals were bred in a rearing room maintained at a room temperature of 20 to 26°C and humidity of 40 to 70% with light and darkness of 12 hours each (illumination: 6:00 a.m. to 6:00 p.m.) and 12 times/hour of air ventilation (fresh air disinfected with a filter). The animals were individually bred by using an aluminum cage (W: 350 × D: 580 × H: 350 mm, provided with an automatic washing apparatus and automatic water supplying apparatus). The amount of feed was 100 g/animal/day for the whole period. As for drinking water, tap water was fed ad libitum by using the automatic water supplying apparatus.

PAA-Pt was diluted with physiological saline and administered into an auricular vein once a day by using a polypropylene disposable syringe (TERUMO) attached with a hypodermic needle (23G, TERUMO). The administration period was 70 days (ten weeks). Before the cholesterol loading, before the start of the administration, and 4, 7 and 10 weeks after the administration, the animals were starved for about 18 hours from the previous day, and about 4 mL of blood was collected from an auricular artery into a blood collecting tube (VP-AS054, TERUMO). For serum obtained by centrifuging the blood (about 4°C, 3000 rpm, 15 minutes) in a refrigerated centrifuge (CF 8 DL, Hitachi Koki), total cholesterol (TC, COD/POD method), triglyceride (TG, GPO/HDAOS method), HDL-C (direct method), and lipid peroxide (LPO, modified Yagi method) were measured.

On the next day of the last day of the administration period, the animals were subjected to abdominal section under anesthetization with pentobarbital sodium (Nembutal Injection, Dainippon Pharmaceutical) and sacrificed by bleeding from the ventral aorta. Then, thoracotomy was carried out, and autopsy findings were recorded. The aorta (from the aortic root to iliac artery) was then extracted, fixed with neutrally buffered 10% formalin, and stained with oil Red. The stained aorta was photographed by using a digital camera (Finepix S1 Pro, Fuji Photo Film), and the ratio of the region stained in red to the total aorta area was calculated on the basis of image analysis. From the obtained numerical values, average ± standard deviation was calculated for each group. The significant difference test was performed between the medium group and each administration group, and a risk factor of less than 5% was considered to be significant. As for the test method, after the results were subjected to the Bartlett's test, Dunnett's test was performed when homoscedasticity was observed, and Steel's test was performed when heteroscedasticity was observed. The average of the arteriosclerosis damaged area (%) in the physiological saline-administered group was 45.5% (p = 13.5). The average of the arteriosclerosis damaged area (%) in the platinum colloid-administered group was 18.7% (p = 6.7) for the 0.005 μ mol/kg administration group, 19.8% (p = 4.0) for the 0.05 μ mol/kg administration group, and 19.2% (p = 4.1) for the 0.5 μ mol/kg administration group.

Industrial Applicability

The medicament of the present invention is useful as a medicament for prophylactic and/or therapeutic treatment of a neurodegenerative disease such as amyotrophic lateral sclerosis and Alzheimer's disease, rheumatic disease such as rheumatoid arthritis, ischemic heart diseases such as myocardial infarction, stress ulcer, dermatitis, arteriosclerosis, and hyperlipidemia.

**Claims**

1. A medicament for prophylactic and/or therapeutic treatment of a disease selected from the group consisting of a neurodegenerative disease, rheumatic disease, ischemic heart disease, stress ulcer, dermatitis, arteriosclerosis, and hyperlipidemia, which comprises as an active ingredient fineparticles of a noble metal or an alloy containing a noble metal.

2. The medicament according to claim 1, wherein the disease is a neurodegenerative disease.

3. The medicament according to claim 2, wherein the neurodegenerative disease is amyotrophic lateral sclerosis, Alzheimer's disease, or Parkinson's disease.

4. The medicament according to claim 1, wherein the disease is a rheumatic disease.

5. The medicament according to claim 4, wherein the rheumatic disease is rheumatoid arthritis.

6. The medicament according to claim 1, wherein the disease is an ischemic heart disease.

7. The medicament according to claim 6, wherein the ischemic heart disease is myocardial infarction at an acute stage.

8. The medicament according to claim 1, wherein the disease is a stress ulcer.

9. The medicament according to claim 8, wherein the stress ulcer is gastric stress ulcer or duodenal stress ulcer.

10. The medicament according to claim 1, wherein the disease is dermatitis.

11. The medicament according to claim 1, wherein the disease is arteriosclerosis.

12. The medicament according to any one of claims 1 to 11, wherein the noble metal is one or more kinds of noble metals selected from the group consisting of ruthenium, rhodium, and platinum.

13. The medicament according to any one of claims 1 to 11, wherein the noble metal is platinum.

14. The medicament according to any one of claims 1 to 13, wherein the fineparticles of a noble metal consist of platinum colloid having a mean particle size of 10 nm or smaller.

Fig. 1

Fig.2

Fig.3

Fig. 4

**Positive**          **PAA-Pt Treatment**

## INTERNATIONAL SEARCH REPORT

| | International application No. |
|---|---|
| | PCT/JP2004/001825 |

**A. CLASSIFICATION OF SUBJECT MATTER**
Int.Cl⁷ A61K33/24, A61P1/04, 3/06, 9/00, 9/10, 17/00, 25/00, 25/16, 25/28, 29/00, 43/00

According to International Patent Classification (IPC) or to both national classification and IPC

**B. FIELDS SEARCHED**

Minimum documentation searched (classification system followed by classification symbols)
Int.Cl⁷ A61K33/24, A61P1/04, 3/06, 9/00, 9/10, 17/00, 25/00, 25/16, 25/28, 29/00, 43/00.

Documentation searched other than minimum documentation to the extent that such documents are included in the fields searched

Electronic data base consulted during the international search (name of data base and, where practicable, search terms used)
CAPLUS, REGISTRY(STN)

**C. DOCUMENTS CONSIDERED TO BE RELEVANT**

| Category* | Citation of document, with indication, where appropriate, of the relevant passages | Relevant to claim No. |
|---|---|---|
| P,X | JP 2003-301288 A (NIPPON TRIM CO., LTD.), 24 October, 2003 (24.10.03), (Family: none) | 1 |
| X | JP 2003-12523 A (OHTSUKA PHARM. IND. CO., LTD.), 15 January, 2003 (15.01.03), (Family: none) | 1-3,12-14 |
| X | JP 2002-241288 A (YAMASHITA SHIRO), 28 August, 2002 (28.08.02), (Family: none) | 1,10,12-14 |
| X | JP 2002-212102 A (AINOBECKS KABUSHIKI KAISHA), 31 July, 2002 (31.07.02), (Family: none) | 1,4,5,8,9, 10,12-14 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22 March, 2004 (22.03.04) | 06 April, 2004 (06.04.04) |

| Name and mailing address of the ISA/ | Authorized officer |
|---|---|
| Japanese Patent Office | |
| Facsimile No. | Telephone No. |

Form PCT/ISA/210 (second sheet) (July 1998)

17

**INTERNATIONAL SEARCH REPORT**

International application No.

PCT/JP2004/001825

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2001-114671 A (OHTSUKA PHARM. IND. CO., LTD.),<br>24 April, 2001 (24.04.01),<br>(Family: none) | 1,10,12-14<br>6-9,11 |
| X<br>Y | JP 2001-10954 A (OHTSUKA SANGYO KABUSHIKI KAISHA),<br>16 January, 2001 (16.01.01),<br>(Family: none) | 1,6-14<br>4,5 |
| Y | JP 2000-232865 A (OHTSUKA PHARM. IND. CO., LTD.),<br>29 August, 2000 (29.08.00),<br>(Family: none) | 1,4-14 |
| Y | JP 11-346715 A (OHTSUKA PHARM. IND. CO., LTD.),<br>21 December, 1999 (21.12.99),<br>(Family: none) | 1,4-14 |
| Y | WO 99/42112 A1 (OHTSUKA PHARM. IND. CO., LTD.),<br>07 September, 1999 (07.09.99),<br>& JP 11-240839 A | 1,4-14 |
| X | JP 11-60493 A (CHIKUCHI EIICHI),<br>02 March, 1999 (02.03.99),<br>(Family: none) | 1,4-11 |
| A | EP 832846 A2 (BASF A.-G.),<br>01 April, 1998 (01.04.98)<br>& DE 19640365 A          & CA 2214441 A<br>& US 5945032 A          & CN 1178232 A<br>& JP 10-110103 A | 1 |
| A | WO 98/14199 A1 (BASF A.-G.),<br>09 April, 1998 (09.04.98),<br>& DE 19640364 A          & EP 939641 A<br>& CN 1238695 A          & US 6231848 B1<br>& JP 2001-501615 A | 1 |
| A | JP 3-243638 A (TODA KOGYO CORP.),<br>30 October, 1991 (30.10.91),<br>(Family: none) | 1 |
| A | JP 2002-60805 A (CHEMIPRO KASEI KABUSHIKI KAISHA),<br>28 February, 2002 (28.02.02),<br>(Family: none) | 1 |
| Y | WO 01/076572 A2 (BITOP GESELLSCHAFT FUER BIOTECHNISCHE OPTIMIERUNG M.B.H.),<br>18 October, 2001 (18.10.01),<br>& EP 1272201 A2          & JP 2003-531833 A<br>& US 2003/147937 A1 | 1-14 |

Form PCT/ISA/210 (continuation of second sheet) (July 1998)